# EUROPÄISCHE PATENTANMELDUNG

(11) **EP 4 541 271 A1**
(43) Veröffentlichungstag der Anmeldung: **23.04.2025**
(21) Anmeldenummer: 24176504.9
(22) Anmeldetag: 17.05.2024
(51) Int. Cl.: A61B 5/024, A61F 7/02, A61F 7/10, A61F 17/00, A61B 5/01, A61B 5/145, A61B 90/96

(54) **HITZENOTFALLKOFFER**

(30) Priorität: 20.10.2023 DE 202023106084 U
(71) Anmelder: pervormance international GmbH, 89075 Ulm (DE)
(72) Erfinder: Renner, Gabriele, 89075 Ulm (DE); Stein, Sabine, 89075 Ulm (DE)
(74) Vertreter: Frick, Robert

(57) **Zusammenfassung**

Die Erfindung betrifft einen Hitzenotfallkoffer umfassend Elemente zur Kühlung, zum Flüssigkeitsersatz, zur Diagnostik sowie eine Anleitung, wie bei Hitzenotfällen zu verfahren ist.

## Beschreibung

Die Erfindung betrifft einen Hitzenotfallkoffer umfassend Elemente zur Kühlung, zum Flüssigkeitsersatz, zur Diagnostik sowie eine Anleitung, wie bei Hitzenotfällen zu verfahren ist.

Aufgrund der Klimaerwärmung haben immer mehr Menschen mit durch hohe Temperaturen verursachten gesundheitlichen Problemen zu kämpfen. Dazu zählen insbesondere Herz-Kreislaufprobleme, Atemprobleme, Hitzekrämpfe, Hitzekollaps und im Ernstfall Hitzschlag. Die Krankenhauseinweisungen und Todesfälle steigen. Hitzeperioden über 45°C auch in gemäßigten Regionen haben gezeigt, dass Hitzenotfallsituationen meist plötzlich aufgrund nicht immer vorrausehbarer Wettersituationen entstehen und dann nicht nur einzelne, sondern gleich viele Menschen auf einmal in Hitzenotfallsituationen geraten. Dadurch ist der normale Notarztdienst oft überfordert und eine schnelle Versorgung durch professionelle Hilfe oft eingeschränkt. Zudem leidet das ausgebildete Personal (Ärzte, Sanitäter, etc.) selbst unter der Hitze und ist vielfach nicht voll einsatzfähig bzw. leistungsfähig.

Hitze ist bisher, im Vergleich zu anderen Notfallsituationen wie beispielsweise Unfällen, Herz-Kreislauf-Notfällen, Asthmaanfällen, einem anaphylaktischen Schock, oder Hypoglykämie, nicht umfassend beschrieben bzw. als Notfall anerkannt. Es gibt vergleichsweise wenig Aufklärung, Schulung und Ausrüstung. Dies spiegelt sich im Spektrum der existierenden Notfallkoffer bzw. -sets wieder.

Nach DIN gibt es bisher Notfallkoffer mit der Erste-Hilfe-Grundausstattung nach DIN 13157, die folgende Produkte enthalten müssen: Wundschnellverbände, Verbandpäckchen in verschiedenen Größen, Verbandtücher, Fixierbinden, Dreiecktücher, Verbandsschere, Rettungsdecke, Einmalhandschuhe und eine Erste-Hilfe-Anleitung. Weiterhin gibt es Notfallkoffer nach DIN 13232:2011 mit drei Teil-Ausrüstungen Basis, Erwachsene und Kinder. Der Notfallkoffer umfasst Instrumente, Medikamente und Material unter anderem für eine Absaugung und Beatmung, z.B. ein Absauggerät und einen Absaugkatheter, für eine Intubation, z.B. einen Laryngoskopgriff, Tuben und einen Führungsstab, für eine Diagnostik, z.B. ein Blutdruckmessgerät, ein Stethoskop, ein Fieberthermometer, für eine Infusion, z.B. ein Infusionsgerät, ein Hautdesinfektionsmittel, einen Venenstauer, und Verbrauchsmaterial, wie z.B. eine Pinzette, Spritzen, Kompressen und Verbandstücher. Zudem gibt es Betriebsnotfallkoffer nach DIN 13157 und 13169, KFZ-Verbandskasten nach DIN 13164, und Sanitätskoffer mit Inhalt nach DIN 13155:2016-03. Auch Defibrillatoren sind als Notfallausstattung anerkannt und allgegenwärtig.

All diese Notfallkoffer sind für Hitzenotfälle aber nur bedingt geeignet. Spezifische Produkte zur Abkühlung oder Flüssigkeitssubstitution fehlen ebenso, wie an nichtfachkundige Personen gerichtete Anleitungen für den Umgang mit einer Hitzenotfallsituation. Häufig bei Hitze empfohlene Kühlmittel wie Eis oder Kühlpacks, etc. sind aufgrund fehlender Kühltemperatureinrichtungen vor Ort und fehlender größerer Energieversorgung der Notfallkoffer, die für Kühlung von Eis oder Kühlpacks benötigt wird, nicht vorhanden.

Aufgabe der Erfindung ist es, einen speziell für Hitzenotfälle geeigneten Notfallkoffer bereitzustellen, der die bestehende Lücke füllt.

Vor diesem Hintergrund betrifft die Erfindung einen Hitzenotfallkoffer umfassend Elemente zur Kühlung, zum Flüssigkeitsersatz, zur Diagnostik sowie eine Anleitung, wie bei Hitzenotfällen zu verfahren ist.

Der Begriff des "Hitzenotfallkoffers" ist im Rahmen der Erfindung als ein nicht notwendigerweise hartschaliges Behältnis zu verstehen, das speziell für den Umgang mit Hitzenotfällen geeignet ist und die genannten Elemente umfasst. Der Hitzenotfallkoffer kann ein hartschaliges Behältnis wie ein Koffer oder eine Box sein, wobei die Schale aus z.B. Metall, wie etwa Aluminium, oder Kunststoff gefertigt sein kann, aber auch z.B. eine Tasche oder ein Rucksack.

Hitzenotfälle können unter anderem Hitzestress, Hitzekrämpfen, Hitzekollaps, Hitzschlag oder Sonnenstich umfassen. Der Hitzenotfallkoffer kann in Unternehmen, in Behörden, im öffentlichen Raum von Städten und Gemeinden, und in Privathaushalten Verwendung finden. Mit dem Hitzenotfallkoffer kann der Hitzenotfall behandelt werden und in schweren Fällen die Zeit bis zum Eintreffen eines Arztes oder Sanitäters überbrückt werden.

Die Elemente zur Kühlung können ein Kühltuch umfassen. Im einfachsten Fall - aus Kostengründen oder für unterwegs kann das Kühltuch so dimensioniert werden, dass es als Hals- Kopf- oder Nackentuch dient. In der Regel sollte das Kühltuch vorzugsweise so dimensioniert sein, dass es eine wesentliche Körperpartie bedecken kann.

Bevorzugte Längen umfassen 50 cm oder größer, 100 cm oder größer, oder auch 150 cm oder größer. Bevorzugte Breiten umfassen 30 cm oder größer, 50 cm oder größer, oder eines der oben angegebenen Längenmaße.

Vorzugsweise ist das Kühltuch ausgebildet, Wasser aufnehmen und nach und nach wieder von seiner Oberfläche verdunsten zu können.

Im einfachsten Fall kann das Kühltuch zur Erfüllung dieser Funktion aus speziellen Kunststofffasern, sogenannten Funktionsfasern insbesondere Polyester- und/oder Polyamidfasern oder anderen Kunststoffarten ausgebildet sein, die verdunstende Flüssigkeiten insbesondere Wasser absorbieren.

In einer weiteren Ausführungsform kann das Kühltuch zur Erfüllung dieser Funktion ein Textil, also ein Gewebe oder einen Vliesstoff umfassen, der mit einem superabsorbierenden Polymer beladen ist.

Ein derartiges Textil bzw. genauer gesagt das assoziierte superabsorbierende Polymer kann durch die Befeuchtung mit einer verdunstenden Flüssigkeit, vorzugsweise mit Wasser beladen werden, um im Nachgang durch eine fortlaufende Verdunstung eine Kühlwirkung zu erreichen. Bei Wasser kann die Kühlleistung bis zu etwa 660 W·h/l betragen. Diese Kühlleistung wird durch die große Oberfläche und mithin große Verdunstungseffektivität erreicht.

Das Textil kann beispielsweise aus Kunstfasern wie Polyamid und/oder Polyester gefertigt werden. Das superabsorbierende Polymer kann in Form von Partikeln vorliegen, die im Textil verteilt, verklebt oder in anderer Weise eingebracht oder befestigt sind. Bevorzugt ist aber, dass die Textilfasern direkt mit einem superabsorbierenden Polymer beschichtet sind. Dadurch wird eine Klumpenbildung vermieden und es kann eine gleichmäßige Verteilung der Feuchtigkeit über das gesamte Textil erreicht werden. Chemisch gesehen handelt es sich bei dem superabsorbierenden Polymer vorzugsweise um ein quervernetztes Polymer mit polaren Eigenschaften. Beispiele umfassen quervernetzte Varianten von Polyacrylamid, Co-Acrylsäure-Acrylamid, Polyurethan oder Polyvinylpyrrolidon. Diese Polymere können große Mengen an Wasser, teilweise mehr als das 1000fache ihres Eigengewichts an Wasser in ihrem Inneren binden. Sie quellen dabei auf. Das superabsorbierende Polymer bindet das Wasser dabei fest und gibt kein flüssiges Wasser ab. Es kann lediglich unter Aufwand von Wärmeenergie wieder aus dem Polymer verdampft werden. Bevorzugte Ausgestaltungen des Textils können der Beschreibung des Patents EP 2 477 588 B1 entnommen werden.

Die besonderen Vorteile eines solchen Materials umfassen die Fähigkeit, Wasser innerhalb von Sekunden aufzunehmen, was in Hitzenotfällen, die ein schnelles Handeln erfordern, von großer Bedeutung ist. Insbesondere steht oftmals auch kein Bassin oder anderes Behältnis zum Einweichen zur Verfügung. Das Material ist im Verhältnis zu seiner Wasseraufnahmekapazität und Kühlleistung leicht und anschmiegsam, und es läuft nicht aus. Dadurch bleibt das Hitzenotfallopfer trocken. Die Bedienung kann durch nur eine Person erfolgen.

Das Textil kann einseitig oder beidseitig mit einer Gewebeschicht überzogen sein, um diesen vor mechanischer Einwirkung zu schützen und optisch zu kaschieren. In einer Ausführungsform ist vorgesehen, dass an der Oberfläche des Textils einseitig oder beidseitig ein Abstandsgewirk angeordnet ist. Durch die Anordnung eines Abstandsgewirks kann die Luftzufuhr zu dem Textil verbessert werden, unter anderem auch wenn dieses einseitig einem Patienten zugewandt ist.

In einer Ausführungsform kann das Kühltuch bereits im aktivierten, also mit Flüssigkeit und insbesondere Wasser beladenen Zustand im Hitzenotfallkoffer vorliegen. In diesem Fall liegt es vorzugsweise in einer Umhüllung wie beispielsweise einer Kunststoffhülle vor, um eine Verdunstung im Hitzenotfallkoffer zu unterbinden. In dieser Ausführungsform würde man sich im Einsatz den Schritt der Aktivierung mit Flüssigkeit, insbesondere mit Wasser sparen. Eine Umhüllung kann aber auch in Fällen eines nicht voraktivierten Kühltuchs sinnvoll sein, um es von Verschmutzung und anderen Umwelteinflüssen zu schützen.

Das Kühltuch kann auch teilweise, d.h. nicht bis zur Grenze der Aufnahmekapazität, sondern bis beispielsweise zwischen 20%-50% der Aufnahmekapazität voraktiviert sein.

Die Umhüllung kann vakuumiert sein, was die Haltbarkeit erhöhen kann. Gegebenenfalls ist in diesem Fall allerdings erforderlich, das Tuch vor dem Einsatz zu schütteln und mit der Bedeckung des Patienten ein paar Sekunden zu warten, da durch das Öffnen des Vakuums Wärme entstehen kann.

Das Kühltuch kann, unabhängig ob es im aktivierten oder im nicht aktivierten Zustand im Hitzenotfallkoffer vorliegt, desinfiziert sein. Eine Desinfektion macht insbesondere dann Sinn, wenn auch eine keimfreie Verpackung, wie beispielsweise eine eben im Zusammenhang mit der Voraktivierung beschriebene Kunststoffumhüllung vorhanden ist.

Zusätzlich oder an Stelle eines mit einem superabsorbierenden Polymer beladenen Textils kann das Kühltuch auch ein Phasenwechselmaterial (Phase Change Material, "PCM") umfassen. Dies kann insbesondere dann sinnvoll sein, wenn der Hitzenotfallkoffer in einer Umgebung mit einer hohen Luftfeuchtigkeit zum Einsatz kommen soll, wo eine Verdunstung von Wasser aus einem mit einem superabsorbierenden Polymer beladenen Textil langsamer erfolgt. Das Phasenwechselmaterial kann flächig verteilt in einer Umhüllung, beispielweise einer Kunststoffhülle vorliegen. Diese kann in Taschen oder anderen Öffnungen des Textils abnehmbar oder fest verbunden angeordnet sein. Sie kann aber auch separat im Notfallkoffer enthalten sein, um nur bei Bedarf verwendet zu werden. Die Phasenübergangstemperatur sollte unterhalb der Körpertemperatur gewählt sein, gleichzeitig aber hoch genug, dass das Material am Lagerort des Hitzenotfallkoffers nicht selbsttätig die Phase wechselt. Geeignet können vor diesem Hintergrund beispielsweise Phasenübergangstemperaturen zwischen 15°C und 30°C sein.

Alternativ oder zusätzlich zu dem Kühltuch kann der Hitzenotfallkoffer auch Kleidungsstücke mit Kühlfunktion umfassen. Die Kühlfunktion dieser Kleidungsstücke kann in einer Ausführungsform dadurch erreicht werden, dass das Kleidungsstück ein wie im Zusammenhang mit dem Kühltuch beschriebenes Textil beinhaltet. Beispiele umfassen Wadenkühler und Armkühler, jeweils z.B. mit Klettverschluss, eine Weste, oder einen Poncho.

Das Kühltuch und/oder Kleidungsstück mit Kühlfunktion kann in einer Ausführungsform mit einem Thermometer zur Messung beispielsweise der Körperoberflächentemperatur des Patienten ausgestattet sein. Das Thermometer kann auch eine Elektronik aufweisen, um die Daten an eine Elektronik des Hitzenotfallkoffers oder einen externen Kommunikationspartner wie ein SmartPhone zu kommunizieren.

In einer Ausführungsform umfasst der Hitzenotfallkoffer ferner einen Ventilator. Es kann sich um einen batteriebetriebenen Ventilator handeln. Der Ventilator kann dazu verwendet werden, die Verdunstung am aktivierten Kühltuch und/oder Kleidungsstück mit Kühlfunktion zu verstärken, und auch ganz allgemein einen kühlenden Luftzug am Patienten verursachen.

Alternativ oder zusätzlich zum Ventilator kann der Hitzenotfallkoffer auch einen Luftfächer umfassen.

Insbesondere in dem Fall, dass das Kühltuch im nicht aktivierten Zustand vorliegt, oder zur allgemeinen Kühlung, oder für den Fall, dass eine Umhüllung des voraktivierten Kühltuchs undicht war und eine Nachaktivierung erforderlich ist, oder für eine ergänzende Aktivierung eines teilweise voraktivierten Kühltuchs, kann der Hitzenotfallkoffer flüssiges Wasser zur Aktivierung beinhalten. Das Wasser kann in einem geeigneten Behälter wie beispielsweise einer Flasche oder einem Beutel vorliegen. Die Volumina sind in Abwägung von Größe und Gewicht einerseits und Bedarf andererseits zu bestimmen und können beispielsweise von 100 ml bis 2 Liter, vorzugsweise von 500 ml bis 1,5 Liter reichen. In einer Ausführungsvariante kann der Behälter mit einer Verteilungshilfe wie einem Zerstäuber oder dergleichen versehen sein.

Alternativ oder zusätzlich kann der Hitzenotfallkoffer auch Wasser als Getränk umfassen. Die Volumina sind auch hier in Abwägung von Größe und Gewicht einerseits und Bedarf andererseits zu bestimmen und können beispielsweise von 100 ml bis 2 Liter, vorzugsweise von 250 ml bis 1 Liter reichen. Das Trinkwasser kann portioniert oder in einer einzigen Portion bereitgestellt sein. Die Bereitstellung kann in einer Trinkflasche oder auch in Beuteln erfolgen.

Der Hitzenotfallkoffer kann zudem Trinkbecher umfassen, um entweder Trinkwasser aus dem Hitzenotfallkoffer oder Getränke aus anderen Quellen an den Patienten verabreichen zu können. Der Trinkbecher kann einen Trinkschnabel bzw. eine Trinkhilfe umfassen, um die Wasseraufnahme durch geschwächte Personen zu erleichtern.

Die Elemente zur Diagnostik können je nach Einsatzziel des Hitzenotfallkoffers von einfach bis professionell ausgestaltet sein, um einen Hitzenotfall diagnostisch von anderen Notfällen zu unterscheiden. Im Normalfall sollten die Elemente zur Diagnostik mindestens ein Thermometer zur Temperaturmessung am Patienten umfassen. Besonders bevorzugt können in einer Ausführungsform Thermometer sein, die ohne Energiezufuhr auskommen. Beispiele umfassen Stirnthermometer-Streifen.

Weiterhin kann der Hitzenotfallkoffer in einer Ausführungsform ein Elektrolytpulver zur Mischung mit einem im Hitzenotfallkoffer ebenfalls vorhandenen oder extern zu beschaffenden Trinkwasser, oder ein vorgefertigtes Elektrolytgetränk umfassen.

In einer Ausführungsform kann der Hitzenotfallkoffer ein Gerät zur Messung des Pulses, der Sauerstoffsättigung, oder beidem umfassen. Beispiele umfassen ein beispielsweise batteriebetriebenes Finger-Pulsoximeter. Das Gerät kann auch eine Elektronik aufweisen, um die Daten an eine Elektronik des Hitzenotfallkoffers oder einen externen Kommunikationspartner wie ein SmartPhone zu kommunizieren.

Weitere geeignete Elemente bzw. Bestandteile des Hitzenotfallkoffers umfassen Einmalhandschuhe, einen Mundschutz, Spuckbeutel, und Erfrischungstücher.

Neben den genannten hitzespezifischen Elementen für z.B. die Kühlung und Hydratisierung kann der Hitzenotfallkoffer auch weitere Elemente bekannter Notfallkoffer für andere Anwendungsschwerpunkte umfassen. Beispiele umfassen Hilfsmittel zur Blutdruckmessung und/oder Hilfsmittel zur Atemunterstützung.

Die Anleitung soll in einer bevorzugten Ausführungsform einfach und an nicht fachkundiges Personal gerichtet sein. Der Mindestinhalt der Anleitung sollte die Beantwortung der folgenden Fragen beinhalten: Wie erkenne ich einen Hitzenotfall? Was ist zu tun? Reihenfolge der Maßnahmen? Beschreibung der Maßnahmen? Inhalt bzw. Anwendung des Inhaltes des Hitzenotfallkoffers? Die Anleitung kann auf den Hitzenotfallkoffer selbst aufgedruckt sein, oder als Beiblatt im oder am Hitzenotfallkoffer vorliegen. Der Hitzenotfallkoffer bzw. die Anleitung kann auch mit QR-Codes oder anderweitigen Verweisen auf Demonstrationsvideos, Audioanleitungen oder schriftlich im Netz verfügbaren Informationen versehen sein. Unter den Sammelbegriff der Audioanleitungen fällt hier auch das Abspielen eines Musikstücks, das den Rhythmus einer Herzdruckmassage vorgibt. Der Hitzenotfallkoffer kann auch selbst Video- oder Audioausgabemittel aufweisen, um Demonstrationsvideos oder Audioanleitungen auszugeben.

Der Notfallkoffer ist in vielen Fällen nur zur Überbrückung der Zeit bis zum Eintreffen eines Arztes oder Sanitäters dienlich, und kein Ersatz für einen Arzt oder Sanitätspersonal. Daher ist in einer Ausführungsform der Notruf sichtbar in die Anleitung integriert und kann in einer Ausführungsform auch digital bereitgestellt oder automatisch ausgelöst werden.

In einer Ausführungsform ist der Hitzenotfallkoffer mit einer Elektronik ausgestattet, die es erlaubt, im Zusammenspiel mit einer SmartPhone-App oder auch autark unterschiedliche Funktionen auszuüben.

Dazu zählt in einer Ausführungsform das Absetzen eines Notrufs, der entweder automatisch oder auf Betätigung aktiviert werden kann. Gemeinsam mit dem Notruf können direkt auch Daten wie den Standort des Hitzenotfallkoffers oder Messdaten z.B. zu Temperatur und Puls mit übertragen werden. In diesem Zusammenhang kann der Hitzenotfallkoffer ausgebildet sein, um seinen Standort bestimmen zu können.

Weiterhin kann auch eine Funktionsüberprüfung vorgesehen sein. Beispielsweise kann ein Mindestbestand vorgegeben oder individuell definiert werden, und es kann über z.B. eine App gemeldet werden, sobald der Mindestbestand unterschritten ist. Somit ist gewährleistet, dass immer genügend Material im Koffer ist. Auch Haltbarkeitsdaten entsprechender Inhaltsstoffe oder Wartungsintervalle können in der App oder der Elektronik abgelegt sein. Die Elektronik kann ferner dazu ausgebildet sein, autark oder auf Aktivierung durch z.B. eine App eine ggf. regelmäßige Überprüfung des Vorhandenseins des Hitzenotfallkoffers am beabsichtigten Standort und/oder der Einsatzfähigkeit des Hitzenotfallkoffers durchzuführen.

Der Koffer kann auch eine Kamera umfassen, um Bilder vom Notfallort an eine Leitstelle übertragen zu können.

Für alle elektronischen Elemente des Hitzenotfallkoffers, beispielsweise Thermometer, Gerät zur Messung des Pulses und/oder der Sauerstoffsättigung, Ventilator, Elektronik zum Absetzen eines Notrufs, etc. gilt, dass diese sowohl batteriebetrieben sein können, als auch auf alternative Stromquellen wie Solarpaneele zurückgreifen können. Die Batterien oder Solarpaneele können am Hitzenotfallkoffer, also an der hart- oder weichschaligen Umhüllung angeordnet sein, am jeweiligen Element angeordnet sein, oder als separates Element innerhalb des Hitzenotfallkoffers vorliegen.

Zur Identifikation des Hitzenotfallkoffers bzw. zum Datenaustausch kommen vorzugsweise stromlose oder stromsparende Technologien zum Einsatz. Beispiele umfassen RFID oder NFC.

Weitere Einzelheiten und Vorteile der Erfindung ergeben sich aus den nachfolgend beschriebenen Figuren und Ausführungsbeispielen. In den Figuren zeigen:
- **Fig. 1:**: einen exemplarischen Hitzenotfallkoffer im geschlossenen Zustand; und
- **Fig. 2:**: den exemplarischen Hitzenotfallkoffer im offenen Zustand.

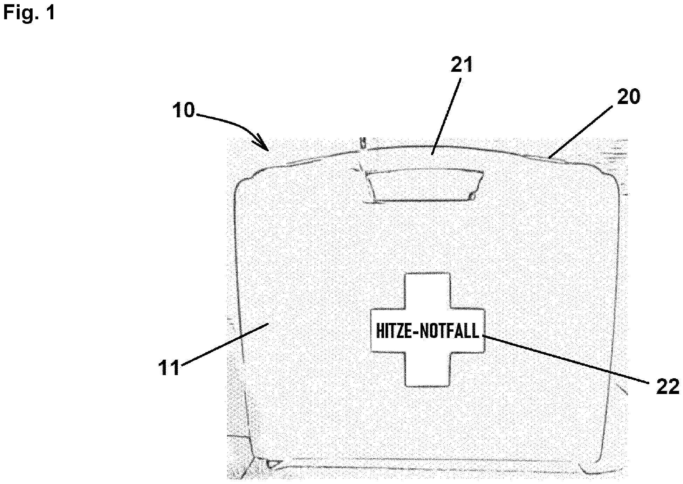

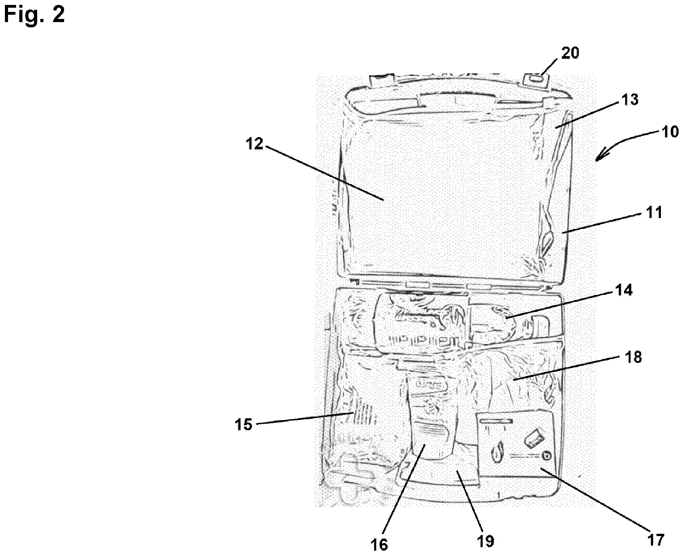

In den Figuren wird exemplarisch ein Hitzenotfallkoffer **10** mit einer Befüllung gemäß dem nachfolgenden Beispiel 1 dargestellt, in der **Fig. 1** in einem geschlossenen und in der **Fig. 2** in einem geöffneten Zustand.

In **Fig. 1** ist zu erkennen, dass der Hitzenotfallkoffer **10** ein Hartschalengehäuse **11** aus Kunststoff umfasst, das die Form eines Koffers mit Einem Griff **21** und zwei seitlich des Griffes **21** angebrachten Schnallen **20** aufweist.

In **Fig. 2** ist der Inhalt des Hitzenotfallkoffers **10** zu erkennen, zumindest in wesentlichen Teilen. Umfasst ist insbesondere ein Kühltuch **12,** das in einer Kunststoffhülle **13** verpackt ist. Weiterhin umfasst ist eine 1-Liter-Flasche **14** mit normalem Wasser, um das Kühltuch **12** aktivieren zu können. Daneben liegen mehrere Beutel **15** mit Trinkwasser sowie Beutel **16** mit einem schnellauflösenden Elektrolyt-Pulver. Letztlich zu erkennen sind ein batteriebetriebenes Finger-Pulsoximeter **17,** in einem Beutel **18** verpackte Einweghandschuhe sowie, etwas verdeckt, eine Anleitung **19.**

### Beispiel 1:

Hitzenotfallkoffer in einer Basisvariante für z.B. Privathaushalte oder kleine Firmen mit dem folgenden Inhalt:
- 1 Kühltuch (z.B. in den Standardmaßen 86 x 52 cm oder 92 x 54 cm). Das Kühltuch ist in Form eines Vliesstoffes ausgebildet, der mit einem superabsorbierenden Polymer beladen ist, wie es im Patent Patents EP 2 477 588 B1 beschrieben wird. Es ist in einer Kunststoffhülle verpackt.
- 1 Liter Wasser zur Aktivierung des Kühltuchs
- 5 Beutel Trinkwasser je 100ml
- 1 Trinkbecher mit Schnabel als Trinkhilfe bei geschwächten Personen
- 5 Beutel schnellauflösendes Elektrolyt-Pulver, je 5 g
- 1 Finger-Pulsoximeter mit Batterien
- 5 Streifen Stirn-Fieberthermometer
- 5 Paar Einmalhandschuhe
- 10 x Mundschutz
- 5 x Spuckbeutel
- 5 x Erfrischungstücher
- 1 Anleitung (Mindestinhalt: Wie erkenne ich einen Hitzenotfall? Was ist zu tun? Reihenfolge der Maßnahmen? Beschreibung der Maßnahmen? Inhalt bzw. Anwendung des Inhaltes des Hitzenotfallkoffers?)

### Beispiel 2:

Hitzenotfallkoffer in einer erweiterten Variante für z.B. Behörden oder größere Unternehmen mit dem folgenden Inhalt:
- 1 Kühltuch (z.B. in den Standardmaßen 86 x 52 cm oder 92 x 54 cm). Das Kühltuch ist in Form eines Vliesstoffes ausgebildet, der mit einem superabsorbierenden Polymer beladen ist, wie es im Patent Patents EP 2 477 588 B1 beschrieben wird. Es liegt in einem mit Wasser zumindest teilweise vorbeladenen Zustand vor und ist in einer vakuumierten Kunststoffhülle verpackt. Das Kühltuch kann ggf. mit einem Thermometer zur Temperaturmessung ausgestattet sein.
- Ggf. 1 oder mehrere Kleidungsstücke mit Kühlfunktion, ausgewählt aus Wadenkühlern und/oder Armkühlern, jeweils ggf. mit Klettverschluss, Westen oder Ponchos.
- 2 Liter Wasser zur (Nach)Aktivierung der Kühltextilien, also von Kühltuch und/oder Kleidungsstück mit Kühlfunktion
- 5 Beutel Trinkwasser je 100ml
- 1 Trinkbecher mit Schnabel als Trinkhilfe bei geschwächten Personen
- 5 Beutel schnellauflösendes Elektrolyt-Pulver, je 5 g
- 1 Finger-Pulsoximeter mit Batterien, ggfs. mit digitaler Schnittstelle
- 5 Streifen Stirn-Fieberthermometer
- Alternativ oder zusätzlich: 1 elektrisches Thermometer, ggf. mit digitaler Schnittstelle
- 5 Paar Einmalhandschuhe
- 10 x Mundschutz
- 5 x Spuckbeutel
- 5 x Erfrischungstücher
- 1 Anleitung (Mindestinhalt: Wie erkenne ich einen Hitzenotfall? Was ist zu tun? Reihenfolge der Maßnahmen? Beschreibung der Maßnahmen? Inhalt bzw. Anwendung des Inhaltes des Hitzenotfallkoffers?)

### Beispiel 3:

Hitzenotfallkoffer für professionelle Anwender wie z.B. Ärzte oder Sanitäter bzw. deren Fahrzeuge mit einem Inhalt wie in Beispiel 2, aber ergänzt um z.B. Hilfsmittel zur Blutdruckmessung und/oder Hilfsmittel zur Atemunterstützung bzw. eines Defibrillators.

## Patentansprüche

1. Hitzenotfallkoffer umfassend Elemente zur Kühlung, zum Flüssigkeitsersatz, zur Diagnostik sowie eine Anleitung, wie bei Hitzenotfällen zu verfahren ist.

2. Hitzenotfallkoffer nach Anspruch 1, wobei Elemente zur Kühlung ein Kühltuch und/oder ein Kleidungsstück mit Kühlfunktion umfassen, das Kühltuch oder Kühltextil vorzugsweise ein Textil in Form eines Vliesstoffs umfasst, der mit einem superabsorbierenden Polymer beladen ist, wobei weiter vorzugsweise die Textilfasern direkt mit einem superabsorbierenden Polymer beschichtet sind.

3. Hitzenotfallkoffer nach Anspruch 2, wobei es sich bei dem superabsorbierenden Polymer um ein quervernetztes Polymer mit polaren Eigenschaften handelt, beispielsweise um Polyacrylamid, Co-Acrylsäure-Acrylamid, Polyurethan oder Polyvinylpyrrolidon.

4. Hitzenotfallkoffer nach einem der Ansprüche 2 oder 3, wobei das Textil einseitig oder beidseitig mit einer Gewebeschicht überzogen ist; und/oder wobei an der Oberfläche des Textils einseitig oder beidseitig ein Abstandsgewirk angeordnet ist.

5. Hitzenotfallkoffer nach einem der Ansprüche 2 bis 4, wobei das Kühltuch oder Kleidungsstück bereits in einem teilweise oder vollständig mit Wasser beladenen Zustand im Hitzenotfallkoffer vorliegt, wobei die teilweise Beladung bei zwischen 20%-50% der Aufnahmekapazität liegen kann.

6. Hitzenotfallkoffer nach einem der Ansprüche 2 bis 5, wobei das Kühltuch oder Kleidungsstück in einer Umhüllung, insbesondere einer Kunststoffhülle vorliegt, wobei die Umhüllung vorzugsweise vakuumiert ist.

7. Hitzenotfallkoffer nach einem der Ansprüche 2 bis 6, wobei das Kühltuch oder Kleidungsstück ein Phasenwechselmaterial umfassen, wobei vorzugsweise das Phasenwechselmaterial flächig verteilt in einer Umhüllung, beispielweise einer Kunststoffhülle vorliegt, und/oder die Phasenübergangstemperatur des Phasenwechselmaterials zwischen 15°C und 30°C liegt.

8. Hitzenotfallkoffer nach einem der vorhergehenden Ansprüche, wobei der Hitzenotfallkoffer ferner einen Ventilator und/oder einen Luftfächer und/oder ein Thermometer zur Temperaturmessung am Patienten und/oder Elektrolytpulver zur Mischung mit Trinkwasser oder ein vorgefertigtes Elektrolytgetränk und/oder Hilfsmittel zur Blutdruckmessung und/oder Hilfsmittel zur Atemunterstützung umfasst.

9. Hitzenotfallkoffer nach einem der vorhergehenden Ansprüche, wobei der Hitzenotfallkoffer flüssiges Wasser in einem oder mehreren Behältern, beispielsweise Flaschen oder Beutel aufweist.

10. Hitzenotfallkoffer nach einem der vorhergehenden Ansprüche, wobei der Hitzenotfallkoffer ein Gerät zur Messung des Pulses, der Sauerstoffsättigung, oder beidem umfasst, insbesondere ein Finger-Pulsoximeter, wobei das Gerät vorzugsweise eine Elektronik aufweist, um Daten an eine Elektronik des Hitzenotfallkoffers oder einen externen Kommunikationspartner zu kommunizieren.

11. Hitzenotfallkoffer nach einem der vorhergehenden Ansprüche, wobei die Anleitung auf den Hitzenotfallkoffer selbst aufgedruckt ist.

12. Hitzenotfallkoffer nach einem der vorhergehenden Ansprüche, wobei der Hitzenotfallkoffer und/oder die Anleitung mit QR-Codes oder anderweitigen Verweisen auf Demonstrationsvideos, Audioanleitungen oder schriftlich im Netz verfügbaren Informationen versehen ist, und/oder wobei der Hitzenotfallkoffer Video- oder Audioausgabemittel aufweisen, um Demonstrationsvideos oder Audioanleitungen auszugeben.

13. Hitzenotfallkoffer nach einem der vorhergehenden Ansprüche, wobei der Hitzenotfallkoffer mit einer Elektronik ausgestattet ist, die ausgebildet ist, den Standort des Hitzenotfallkoffers bestimmen zu können und/oder einen Notruf abzusetzen, wobei die Elektronik vorzugsweise ausgebildet ist, gemeinsam mit dem Notruf Daten wie beispielsweise den Standort des Hitzenotfallkoffers oder Messdaten z.B. zu Temperatur und Puls am Patienten mit zu übertragen.

14. Hitzenotfallkoffer nach einem der vorhergehenden Ansprüche, wobei der Hitzenotfallkoffer mit einer Elektronik ausgestattet ist, die ausgebildet ist, selbst oder in Wechselwirkung mit einem externen Kommunikationspartner eine Funktionsüberprüfung des Hitzenotfallkoffers durchzuführen, entweder automatisch nach beispielsweise der Öffnung des Koffers oder auf Betätigung, wobei die Funktionsüberprüfung vorzugsweise eine Überprüfung beinhaltet, ob genügend Material im Hitzenotfallkoffer ist oder ob der Hitzenotfallkoffer am beabsichtigten Standort ist.

15. Hitzenotfallkoffer nach einem der vorhergehenden Ansprüche, wobei der Hitzenotfallkoffer mit einer Elektronik ausgestattet ist und eine Kamera umfasst, wobei die Elektronik ausgebildet ist, mit der Kamera aufgenommene Bilder an einen externen Kommunikationspartner zu übertragen.
